# EUROPEAN PATENT SPECIFICATION

(11) **EP 3 780 975 B1**
(45) Date of publication and mention of the grant of the patent: **10.08.2022**
(21) Application number: 18720172.8
(22) Date of filing: 17.04.2018
(51) Int. Cl.: A23L 33/105, A23L 29/00, A61P 39/06, A61K 36/37, A61K 36/53, A61K 31/191, A61K 31/216, A23L 27/00, A23L 2/39, A61K 36/30

(54) **SUSTAINED ENERGY PROVIDING AND FREE RADICAL PROTECTIVE COMPOSITION**
ZUSAMMENSETZUNG MIT ANHALTENDER ENERGIEBEREITSTELLUNG UND SCHUTZ VOR FREIEN RADIKALEN
COMPOSITION ASSURANT UNE PROTECTION CONTRE LES RADICAUX LIBRES ET FOURNISSANT UN APPORT ÉNERGÉTIQUE DURABLE

(43) Date of publication of application: 24.02.2021
(73) Proprietor: Protea Biopharma N.V., 1120 Bruxelles (BE)
(72) Inventor: ROELANT, Christiaan Hubert Simon, 3000 Leuven (BE); DE MEIRLEIR, Kenny Leo, 2800 MECHELEN (BE)
(74) Representative: Tomic, Marija
(86) International application number: PCT/EP2018/059786
(87) International publication number: WO 2019/201430

(56) References cited:
- WO-A1-2008/055651
- WO-A1-2015/196099
- WO-A2-2008/090474
- JP-A- 2004 315 409
- JP-A- 2008 214 250
- US-A1- 2011 263 697
- BORS W ET AL: "Antioxidant mechanisms of polyphenolic caffeic acid oligomers, constituents of Salvia officinalis", BIOLOGICAL RESEARCH 2004 CL, vol. 37, no. 2, 2004, pages 301-311, XP9510182, ISSN: 0716-9760
- BRUNA DE FALCO ET AL: "Chia seeds products: an overview", PHYTOCHEMISTRY REVIEWS, vol. 16, no. 4, 9 May 2017 (2017-05-09), pages 745-760, XP055536869, NL ISSN: 1568-7767, DOI: 10.1007/s11101-017-9511-7

## Description

### Technical field:

A natural product- based sustainable energy composition simultaneously providing protection against toxic free radicals.

### Prior art:

Energy providing compositions that are used to overcome tiredness are based on high caffeine and high sugar content supplemented with high dose vitamin and electrolyte concentrations. These are sold as energy drinks under brand names such as Red Bull, Monster, Rockstar, Powerade, and Gatorade. The drinks or beverages provide instant energy and may facilitate human performance over a limited period of time but with a serious risk of free radical generation and tissue damage.

### Summary of the invention:

The invention relates to compositions providing natural product-based sustainable energy in combination with free radical neutralization and method of use thereof.

### Detailed description of the preferred embodiments:

Instant energy providing compositions usually contain high concentrations of monomeric sugars such as glucose, fructose and galactose in combination with caffeine and therefore are effective to combat fatigue over a short period of time. The composition of such oral consumables however may have detrimental effects due to overworking tissues and raising toxic free radicals in the body. In order to improve the sustained energy release process, compositions have been suggested such as described in EP 1 712 139 A1 describing the use of glucopyranosyl sugar alcohols or the use of specific plant extracts as claimed in US 8,642,097 B1. Prior art compositions however solely focus on sustained energy delivery without providing any solution to the problem that metabolically forced tissue activity may lead to dramatic increases of toxic radical production, in particular reactive oxygen species, that may be extremely harmful. Especially up-growing younger people or people with a weak health condition trying to physically improve by consuming energy drinks may become prone to the noxious side effects caused by energy drinks, in particular the production of toxic radicals which affect heart function and ultimately may lead to conditions of cancer, brain damage, faster aging and others. US2011/263697(A) and JP2004 315409(A) discuss the effects of caffeic acid oligomers, notably rosmarinic acid.

In order to overcome prior art disadvantages, the invention proposes the use of compositions using products that at the same time are energy donors over an extended period of time as well as neutralizing agents of toxic substances, in particular free radicals.

The prolonged and sustained release of energy is obtained through metabolization of the oligomeric form of product into monomers. For this purpose, oligomeric forms of sugars have been described in the prior art. In contrast, the invention describes the use of dimers, trimers and oligomers that are no-sugar oligomers but poly-oxyphenols and of which rosmarinic acid is the preferred type. Rosmarinic acid is metabolized into caffeic acid and salvianic acid. The caffeic acid is a polyphenol, a class of micronutrients known for their antioxidative properties. Despite of its name, it is unrelated to caffeine. Caffeic acid is claimed to: reduce inflammation, to prevent cancer, to prevent diabetes, to prevent premature aging, to prevent neurodegenerative diseases like Parkinson, to potently inhibit amylin amyloidosis ( a suspected disease pathway in the onset of Alzheimer disease), to protect and to attenuate cardiovascular disease, to have anti-microbial activity and to reduce exercise-related fatigue. Additionally it mitigates oxidative diabetes-induced brain damage and protects pancreatic cells from glucolipotoxicity.

Rosmarinic acid is found in plant families such as the *Lamiaceae, Boraginaceae* and *Celestraceae.* Therefore, ideally, water-extracts of leaves of plants containing monomers, dimers, trimers and oligomers of Rosmarinic acid constitute an excellent source of sustained energy provider, because of the slow metabolisation into caffeic acid ( heart, muscles, brain e.g.) and free radical scavenger protecting tissues from being damaged by toxic radicals.

In the claimed invention, the Rosmarinic acid is obtained from the dried leaves of a plant known as *Celastrus hindsii Benth* by extraction from the dried leaves with hot/boiling water to form a water extract or tea. In addition to the monomer and oligomers of Rosmarinic acid, the leaves of this plant also contain Rutin and Lithospermic acid. Rutin is a flavonoid with general anti-oxidant properties and supports blood vessel health. Lithospermic acid is a dimeric form of Rosmarinic acid with higher afinity to silver (Ag⁺) ions.

In another preferred embodiment, the highly concentrated water extract prepared from the dried leaves, containing rosmarinic acid monomers, -dimers, -trimers and oligomers is further concentrated by freeze-drying. Hereto equal volumes of water and compact dried leaves are admixed. The admixture is boiled at the stage where the volume of leaves becomes reduced and the boiling water fraction has a tendency to rise. Next, the boiling process is stopped and the admixture left to cool down before being poured through a filter or cloth to separate the solid fraction of boiled leaves from the liquid dark colored extract.

The extract can be stored in a fridge and used as a concentrated liquid extract to prepare a drink or to add to other beverages ( any non-alcoholic as well as alcoholic ) or energy drinks to improve energy delivery over time and to reduce possible risks of tissue injury as may be obtained by consuming regular energy drinks.

In a further embodiment the water extract is being processed as a freeze-dried or hot-sprayed solid form either ready for use to prepare an instant drink by adding a liquid (non-alcoholic as well as alcoholic) or as a solid powder that can be mixed with or added to non-limited list of probiotics, sauces, solid foods, cream, gels, cosmetics, perfumes, ointments, spices, flavoring, seasonings. The freeze-dried extract can be stored with a shelf life of at least 5 years in a dry environment at ambient temperature.

## Claims

1. Use of caffeic acid oligomeric forms in the preparation of a sustained energy release food, feed, nutraceutical and/or beverage composition simultaneously providing maximal tissue protection by neutralization of a broad spectra of free radicals associated with energy consuming metabolic reactions, wherein the dried leaves of Celastrus hindsii Benth are used to directly prepare a water extract as a tea or the basis of a cold beverage.

2. Use according to claim 1, wherein the water extract is freeze- or spray dried and the resulting product can be stored or further processed as an instant soluble powder that can be added to or mixed with any food, beverage or sustained energy-release requiring material such as nutraceuticals, cosmetics and the like.

3. Use according to claim 2, and to add to improve the composition of existing energy- providing beverages, foods, cosmetics and the like.

4. A composition for use as a tea or as the basis of a cold beverage, the composition comprising caffeic acid oligomeric forms in an aqueous extract of dried leaves of Celastrus hindsii Benth, and optionally, at least one further beverage component.

5. The composition according to claim 4, as a freeze dried or hot-sprayed free flowing powder.

6. The composition according to claim 4 or 5, further comprising a probiotic, a sauce composition, a solid food composition, a cream, gel ointment base, a cosmetic or personal care composition, and/or one or more perfumes, flavorings, spices and/or seasonings.

7. A process for the preparation of an instant beverage, comprising
(a) providing the composition according to any one of claims 4 to 6 as a freeze-dried or hot-sprayed solid form, and
(b) adding a non-alcoholic or alcoholic liquid, to obtain the beverage composition.

## Patentansprüche

1. Verwendung von Coffeinsäure-Oligomerformen bei der Herstellung einer Lebensmittel-, Futtermittel-, Nutrazeutikum- und/oder Getränkezusammensetzung mit anhaltender Energiefreisetzung, die gleichzeitig größtmöglichen Gewebeschutz durch Neutralisation eines breiten Spektrums von freien Radikalen, die mit energieverbrauchenden Stoffwechselreaktionen verbunden sind, bereitstellt, wobei die getrockneten Blätter von Celastrus hindsii Benth zur direkten Herstellung eines Wasserextrakts als Tee oder die Grundlage eines Kaltgetränks verwendet werden.

2. Verwendung gemäß Anspruch 1, wobei der Wasserextrakt gefrier- oder sprühgetrocknet wird und das erhaltene Produkt gelagert oder zu einem sofortlöslichen Pulver weiterverarbeitet werden kann, das zu/mit einem beliebigen Lebensmittel, Getränk oder Material, das anhaltende Energiefreisetzung erfordert, wie z. B. Nutrazeutika, Kosmetika und dergleichen, zugegeben oder gemischt werden kann.

3. Verwendung gemäß Anspruch 2 und zum Zugeben zum Verbessern der Zusammensetzung von bestehenden energiebereitstellenden Getränken, Lebensmitteln, Kosmetika und dergleichen.

4. Zusammensetzung zur Verwendung als Tee oder als die Grundlage eines Kaltgetränks, wobei die Zusammensetzung Coffeinsäure-Oligomerformen in einem wässrigen Extrakt von getrockneten Blättern von Celastrus hindsii Benth und gegebenenfalls wenigstens eine weitere Getränkekomponente umfasst.

5. Zusammensetzung gemäß Anspruch 4 als gefriergetrocknetes oder heißgesprühtes, frei fließendes Pulver.

6. Zusammensetzung gemäß Anspruch 4 oder 5, ferner umfassend ein Probiotikum, eine Saucenzusammensetzung, eine feste Lebensmittelzusammensetzung, eine Creme, eine Gelsalbengrundlage, eine kosmetische oder Körperpflegezusammensetzung und/oder ein oder mehrere Parfüme, Aromastoffe, Gewürze und/oder Würzmittel.

7. Verfahren zur Herstellung eines Instantgetränks, umfassend
(a) Bereitstellen der Zusammensetzung gemäß einem der Ansprüche 4 bis 6 als gefriergetrocknete oder heißgesprühte feste Form und
(b) Zugeben einer nichtalkoholischen oder alkoholischen Flüssigkeit, um die Getränkezusammensetzung zu erhalten.

## Revendications

1. Utilisation de formes oligomériques d'acide caféique dans la préparation d'une composition d'aliment, d'aliment pour animaux, nutraceutique et/ou de boisson, à libération d'énergie prolongée fournissant simultanément une protection tissulaire maximale par neutralisation d'un large spectre de radicaux libres associés à des réactions métaboliques consommant de l'énergie, les feuilles séchées de Celastrus hindsii Benth étant utilisées pour préparer directement un extrait aqueux comme un thé ou la base d'une boisson froide.

2. Utilisation selon la revendication 1, l'extrait aqueux étant lyophilisé ou séché par pulvérisation et le produit résultant pouvant être stocké ou traité davantage en tant que poudre soluble instantanée qui peut être ajoutée ou mélangée avec un(e) quelconque aliment, boisson ou matière requérant la libération d'énergie prolongée telle que des substances nutraceutiques, des cosmétiques et autres.

3. Utilisation selon la revendication 2, et pour être ajoutée pour améliorer la composition de boissons, d'aliments, de cosmétiques fournissant de l'énergie existants et autres.

4. Composition pour une utilisation en tant que thé ou comme la base d'une boisson froide, la composition comprenant des formes oligomériques d'acide caféique dans un extrait aqueux de feuilles séchées de Celastrus hindsii Benth, et éventuellement, au moins un autre composant de boisson.

5. Composition selon la revendication 4, en tant que poudre s'écoulant librement lyophilisée ou pulvérisée à chaud.

6. Composition selon la revendication 4 ou 5, comprenant en outre un probiotique, une composition de sauce, une composition alimentaire solide, une crème, une base d'onguent en gel, une composition de soin cosmétique ou personnel, et/ou un(e) ou plusieurs parfums, arômes, épices et/ou assaisonnements.

7. Procédé pour la préparation d'une boisson instantanée, comprenant
(a) la mise à disposition de la composition selon l'une quelconque des revendications 4 à 6 sous une forme solide lyophilisée ou pulvérisée à chaud, et
(b) l'ajout d'un liquide non alcoolique ou alcoolique, pour obtenir la composition de boisson.
